# FASCICULE DE BREVET EUROPEEN

(11) **EP 1 658 043 B1**
(45) Date de publication et mention de la délivrance du brevet: **14.12.2011**
(21) Numéro de dépôt: 04767367.8
(22) Date de dépôt: 17.06.2004
(51) Int. Cl.: A61K 8/64, A61K 8/97, A61Q 19/08

(54) **EXTRAIT DE MACA ET COMPOSITION COSMETIQUE COMPRENANT UN TEL EXTRAIT**
MACA-EXTRAKT UND DIESEN EXTRAKT ENTHALTENDE KOSMETISCHE ZUSAMMENSETZUNG
MACA EXTRACT AND COSMETIC COMPOSITION CONTAINING SUCH AN EXTRACT

(30) Priorité: 19.06.2003 FR 0307388
(43) Date de publication de la demande: 24.05.2006
(73) Titulaire: Laboratoires Expanscience, 92400 Courbevoie (FR)
(72) Inventeur: PICCARDI, Nathalie, F-21310 Arceau (FR); PICCIRILLI, Antoine, F-78000 Versailles (FR); MSIKA, Philippe, F-78000 Versailles (FR); PAUL, François, F-31400 Toulouse (FR)
(74) Mandataire: Warcoin, Jacques
(86) Numéro de dépôt international: PCT/FR2004/001505
(87) Numéro de publication internationale: WO 2004/112742

(56) Documents cités:
- FR-A- 2 778 565
- FR-A- 2 802 418
- GB-A- 2 011 910
- PATENT ABSTRACTS OF JAPAN vol. 2003, no. 09, 3 septembre 2003 (2003-09-03) & JP 2003 155213 A (SHIYAROONE:KK), 27 mai 2003 (2003-05-27) -& DATABASE WPI Section Ch, Week 200377 Derwent Publications Ltd., London, GB; Class B04, AN 2003-818504 XP002309876 -& JP 2003 155213 A (SHARONE KK) 27 mai 2003 (2003-05-27)
- COMAS M ET AL: "ESTUDIO BROMATOLOGICO DE LA MACA O PACA (LEPIDIUM MEYENII)" ALIMENTARIA, MADRID, ES, octobre 1997 (1997-10), pages 85-90, XP002942756 ISSN: 0300-5755 cité dans la demande
- PATENT ABSTRACTS OF JAPAN vol. 2000, no. 19, 5 juin 2001 (2001-06-05) & JP 2001 039854 A (SANKO BUSSAN KK), 13 février 2001 (2001-02-13) -& DATABASE WPI Section Ch, Week 200130 Derwent Publications Ltd., London, GB; Class B04, AN 2001-285641 XP002309877 -& JP 2000 039854 A (SANKO BUSSAN KK) 8 février 2000 (2000-02-08)
- PATENT ABSTRACTS OF JAPAN vol. 2000, no. 14, 5 mars 2001 (2001-03-05) & JP 2000 319120 A (ICHIMARU PHARCOS CO LTD), 21 novembre 2000 (2000-11-21) -& DATABASE WPI Section Ch, Week 200110 Derwent Publications Ltd., London, GB; Class D21, AN 2001-084830 XP002309878 -& JP 2000 319120 A (ICHIMARU PHARCOS INC) 21 novembre 2000 (2000-11-21)
- PARRADO J ET AL: "PRODUCTION OF SOLUBLE ENZYMATIC PROTEIN HYDROLYSATE FROM INDUSTRIALLY DEFATTED NONDEHULLED SUNFLOWER MEAL" JOURNAL OF AGRICULTURAL AND FOOD CHEMISTRY, AMERICAN CHEMICAL SOCIETY. WASHINGTON, US, vol. 39, no. 3, 1 mars 1991 (1991-03-01), pages 447-450, XP000217411 ISSN: 0021-8561

## Description

La présente invention concerne un procédé d'extraction de maca, l'extrait obtenu, une composition cosmétique le contenant ainsi que son utilisation à titre d'agent cosmétique anti-âge.

Le nom botanique de la maca est le *Lepidium meyenii Walp.* Parmi ses noms vernaculaires on peut encore citer en anglais : maca, peruvian ginseng, quechua ; en espagnol : maca, maka, maca-maca et en kechua : ayak chichica, ayak willku, maka. Elle appartient à la famille des Brassicaceae (Cruciferae) de la Tribu Lepidieae.

La maca est une petite plante herbacée de 12 à 20 cm de haut. Sa partie souterraine mesure de 2 à 5 cm. Elle comprend une racine pivot surmontée de la partie basse d'un hypocotyle élargi et charnu. A l'état sec, l'ensemble rappelle la forme d'un petit navet. Pour simplifier, la partie souterraine de la plante qui constitue la fraction employée sera dénommée "tubercule".

Les feuilles forment une rosette et se renouvellent depuis son centre. Les petites fleurs sont autogames. Le fruit est une petite silique (4 à 5 mm) à deux valves comprenant chacune une graine.

La Maca et les autres Lepidium sauvages botaniquement proches sont localisées jusqu'à présent dans quelques zones montagneuses de la cordillère des Andes (Pérou, Bolivie, Equateur). Ces plantes sont capables de supporter des gelées même au cours de leur période de croissance. Considérées longtemps comme des plantes "à jour court" en raison de leur habitat, des travaux relatifs à leur photopériodisme ont révélé que leur croissance est similaire dans des conditions de jours courts et de jours longs.

La plante présente un comportement annuel lorsque les conditions climatiques lui sont favorables (sol suffisamment humide et température tempérée). Son cycle végétatif est alors de 11 mois. Elle devient bisannuelle en climat de haute montagne en conservant sa partie souterraine en dormance pendant la saison sèche.

La Maca fut probablement "domestiquée" à San Blas au Pérou, il y a 1300 à 2000 ans. Depuis, sa culture a toujours été confinée aux montagnes centrales du Pérou entre 3 500 et 4 500 mètres d'altitude dans les départements du Junin et du Pasco. Les zones de culture les plus importantes sont concentrées autour du lac de Junin. Moins restreintes autrefois, elles s'étendaient jusqu'à Cusco et le lac Titicaca. Dans ces contrées, les basses températures et les vents violents limitent fortement d'autres cultures en dehors de la pomme de terre.

La maca est actuellement cultivée dans de petites parcelles de 500 m² selon des méthodes très artisanales. Les graines sont semées au début de la période des pluies en septembre-octobre. Les tubercules sont habituellement récoltés 8 à 10 mois après semis. La récolte commence en mai-juin. Après récolte, les tubercules sont laissés sécher au soleil pendant 6 à 15 jours. Ils seront conservés à l'abri de la lumière et de l'humidité en attendant d'être consommés. Les tubercules se conservent bien.

Les principaux résultats d'analyse de la composition chimique de la maca ont été publiés par Dini et al. en 1994 (Dini A., Migliuolo G., Rastrelli L., Saturnino P., Schettino O. Chemical composition of Lepidium meyenii. Food chemistry, 1994, 49, 4, pp. 347-349 (eng)) puis par Comas et al. en 1997 (Comas M., Miquel X., Arias G., de la Torre M.C. Bromatological studies on Lepidium meyenii. Alimentaria (Madrid), 1997, 286, pp. 85-90 (spa)) :
- humidité : 10 à 20 %
- matières minérales les plus intéressantes (mg/100 g) :
   - Potassium 1150 à 2050
   - Calcium 150 à 260
   - Fer 3 à 16
   - Cuivre 0.2 à 6
   - Zinc 1.5 à 6
   - Aluminium 3 à 7
- Glucides : 60 à 65 %
   - amidon 30 à 35 %
   - Saccharose 3 à 20 %
   - Fructose 8 à 10 %
- Glucose 3 à 7 %
- Fibres : 4 à 8 %
- Protéines: 10 à 14 %
- Lipides : 0,5 à 2 %

La maca est traditionnellement utilisée comme aliment, mais aussi pour ses propriétés thérapeutiques.

La valeur nutritive du tubercule de la maca, proche de céréales classiquement utilisées en alimentation, en fait un aliment de choix et d'intérêt majeur pour les populations de haut plateaux péruviens.

Le tubercule de la maca est employé depuis des centaines d'années en usage populaire à des fins médicinales pour augmenter la fertilité des animaux et des êtres humains (Leon J., The maca (Lepidium meyenii), a little-known food plant of Peru. Economic botany, 1964, 18, 2 pp. 122-127 (eng)).

Les Kallawaya, guerisseurs itinérants des Andes, prescrivaient aux femmes stériles désirant être fécondées, le tubercule frais découpé en fines rondelles, en décoction, trois ou quatre jours après les dernières règles (Girault L. Kallawaya. Guérisseurs itinérants des Andes. ORSTOM éd., Paris, 1984, pp. 218-219 (fra)).

De nos jours, la popularité de la maca s'accroît en raison des propriétés stimulantes et aphrodisiaques qui lui sont attribuées. Le tubercule de la maca est apparenté (abusivement en raison d'un marché potentiel prometteur), au ginseng (Panax ginseng) d'où son nom de ginseng péruvien.

Parmi les autres utilisations du tubercule figurent son intérêt en cas de troubles respiratoires (tuberculose), fatigue chronique, troubles de la mémoire, symptômes de la ménopause, en cures lors de crises rhumatismales, etc

Le but de la présente invention est de proposer un extrait de maca permettant de stimuler le métabolisme et la prolifération des fibroblastes pour prévenir et/ou lutter contre le vieillissement cutané chronologique, extrinsèque (soleil, tabac, pollution, stress) et ménopausique.

En effet, le vieillissement cutané est notamment caractérisé par une diminution du nombre, ainsi que de l'activité des fibroblastes.

En effet, la maca brute, qui se présente généralement sous la forme d'une poudre déshydratée, est quasiment insoluble dans l'eau. De ce fait, son utilisation dans les produits de soins cosmétiques est difficilement envisageable en l'état. Par ailleurs, la biodisponibilité des molécules constitutives du végétal (sels minéraux, glucides, protéines, vitamines, ...) est quasi nulle par voie cutanée.

Ainsi l'invention concerne un extrait peptidique de maca, totalement hydrosoluble, susceptible d'être obtenu par le procédé selon la revendication 1 son procédé d'obtention, les compositions cosmétiques le contenant ainsi que leur utilisation en tant qu'actif anti-vieillissement.

L'extrait peptidique peut être liquide ou solide suivant si l'extrait a subi une lyophilisation ou non en deuxième étape du procédé d'obtention.

Le vieillissement cutané peut se manifester notamment par l'affaissement des tissus, ce qui peut en particulier se traduire par une perte de tonicité et de fermeté de la peau, par la diminution de l'épaisseur et de l'élasticité de la peau, par l'apparition de taches pigmentaires de sénescence et par une perte d'éclat et d'uniformité de la peau ou encore par l'apparition de rides ou ridules.

L'invention a pour objet un procédé de préparation d'un extrait peptidique aqueux de maca, selon la revendication 1 caractérisé en ce qu'il est effectué à partir de farine de tubercules de maca broyés, en ce qu'il comprend au moins une étape d'hydrolyse enzymatique des protéines.

L'hydrolyse enzymatique est menée avec un mélange amylase et protéase. De préférence, le ratio amylase/protéase est compris entre 50/50 et 90/10 de préférence entre 75/25 et 85/15 afin de transformer la fraction protéique du végétal, en peptides hydrosolubles.

Selon le procédé de l'invention, l'extrait aqueux est ensuite purifié par ultrafiltration afin d'extraire les éventuelles traces de protéines résiduelles. Dans ce cas, on choisira avantageusement un seuil de coupure de 10 kD de sorte à conserver les peptides présentant un poids moléculaire inférieur à 10 kD.

Ainsi, selon une variante préférée de l'invention, le procédé comprend les étapes suivantes:
- un lavage et un séchage sous courant d'air chaud, (par exemple à 60°C) des tubercules de maca,
- le broyage des tubercules de maca en une farine fine,
- la mise en suspension dans l'eau de la farine, avantageusement entre 1 et 25% en poids,
- l'hydrolyse des protéines en présence d'une protéase et d'une amylase par exemple dans un rapport 80/20.
- une centrifugation pour éliminer les insolubles (fibres),
- une étape d'ultrafiltration de la solution (avantageusement seuil de coupure 10 kD),
- suivie par une étape de concentration en matière sèche par diafiltration (avantageusement 100 Da) et/ou une étape d'évaporation contrôlée,
et enfin éventuellement suivie par une étape de filtration stérilisante (préférentiellement sur une cartouche de 0,2 µm).

L'invention a en outre pour objet un extrait peptidique aqueux de maca susceptible d'être obtenu par le procédé selon la revendication 1.

Cet extrait peptidique aqueux de maca présente une teneur en matière sèche comprise entre 1 et 300 g/l, de préférence entre 2 et 10 g/l.

Par rapport à la matière sèche, la teneur en sucres réducteurs est comprise entre 35 et 45 %. Par sucres réducteurs on entend des sucres réactifs: ils ont la faculté de donner des électrons à une molécule. On peut citer le glucose, le fructose et le maltose. Historiquement, ce terme vient de la découverte de Fehling au 19^{ème} siècle qui prouva que certains sucres réagissaient avec des ions cuivriques pour les transformer en ions cuivreux. Visuellement, cette réaction dite « de réduction » s'observe par un changement de couleur de la liqueur de Fehling : au départ bleue, elle vire au rouge brique en présence de sucres réducteurs.

Le pH d'une solution à 20 g/l de matière sèche pourra être compris entre 5 et 8, de préférence entre 6 et 7.

De plus, l'invention a pour objet un procédé de préparation d'un extrait peptidique solide de maca, caractérisé en ce que l'extrait peptidique aqueux., éventuellement stérilisé est lyophilisé. On obtient autrement dit une poudre solide (extrait sec), qui présente notamment l'avantage d'être hydrosoluble, ce qui n'est pas le cas de la farine de tubercule de maca originelle.

L'invention a aussi pour objet un extrait peptidique solide de maca susceptible d'être obtenu par le procédé selon la revendication 1.

Cet extrait peptidique solide de maca est en outre caractérisé par sa teneur en azote alpha aminé comprise entre 2 et 70%.

De préférence, l'extrait peptidique solide de maca selon l'invention présente la composition en acides aminés suivantes (en pourcentage en poids par rapport au poids total d'acides aminés) :

| | |
|---|---|
| Alanine | 5-9 % |
| Arginine | 15-20 % |
| Acide aspartique | 8-12 % |
| Cystine-cysteine | < 2 % |
| Acide glutamique | 9-15 % |
| Glycine | 3-7 % |
| Histidine | 1-6 % |
| Isoleucine | 2-7 % |
| Leucine | 4-9 % |
| Lysine | 3-7 % |
| Methionine | 1-5 % |
| Phenylalanine | 4,9 |
| Proline | < 1 % |
| Sérine | 2-8 % |
| Thréonine | 1-7 % |
| Tyrosine | 1-7 % |
| Valine | 4-10 % |
| Tryptophane | < 0,5 % |

L'invention a également pour objet une composition cosmétique caractérisée en ce qu'elle comprend un extrait peptidique aqueux ou solide de maca tel que décrit précédemment et au moins un excipient cosmétiquement acceptable.

Une telle composition cosmétique peut être notamment destinée à lutter contre le vieillissement cutané. L'invention a donc aussi pour objet une méthode de traitement cosmétique comprenant l'application d'une telle composition sur la surface cutanée d'un individu. Enfin, l'invention a pour objet l'utilisation d'un extrait peptidique aqueux ou solide selon l'invention en tant qu'actif anti-vieillissement. Plus particulièrement, cet extrait aqueux ou solide peut être utile pour stimuler le métabolisme cellulaire à savoir l'activité mitochondriale et notamment les fibroblastes dermiques. De même, cet extrait aqueux ou solide peut être utile pour stimuler l'énergie cellulaire. Par « énergie cellulaire » on entend le réservoir d'énergie dans lequel la cellule puise pour réaliser l'ensemble de ses activités vitales (notamment mitose, croissance, synthèse des macromolécules, réparation de l'ADN). Enfin il peut être utile pour lutter contre les agressions extérieures de type soleil, tabac, pollution ou stress.

L'invention est maintenant illustrée par les exemples de réalisation décrits ci-après.

### EXEMPLE 1: préparation d'un extrait

10 kg de farine de maca sont dispersés dans 80 litres d'eau déminéralisée, en présence de 0,25 kg d'amylase.

Le mélange est maintenu à 50 °C, pendant 5 heures, à pH constant de 5.

Dans une seconde étape, 0,25 kg de protéase Alcalase® commercialisé par la société Novo Nordisk sont ajoutés. Le mélange est alors maintenu à 60°C, pendant 1 heure, à pH constant de 8.

Les enzymes hydrolytiques sont ensuite dénaturées par chauffage à 90°C, pendant 20 minutes.

Le mélange est centrifugé à 5500 tr/min en présence d'un adjuvant argileux de filtration puis filtré sur des toiles de 1 µm pour être clarifié.

Le solution récupérée est alors ultrafiltrée (seuil de coupure 10 kD), le filtrat concentré par diafiltration (10 Da) jusqu'à un titre de 10 % en matière sèche, puis filtrée stérilement (0,2 µm).

L'extrait obtenu présente les caractéristiques suivantes :

| | |
|---|---|
| Aspect / Couleur | Solution limpide de coloration jaune |
| Odeur | Caractéristique |
| Matière sèche (p/p) | 10,4 % |
| pH en solution à 20g/l | 6,8 |
| Absorbance | 0,530 à 420 nm |
| | 0,093 à 550 nm |
| **Composition par rapport à la matière sèche (p/p)** | |
| Azote alpha aminé | 4 % |
| Azote total | 1,7 % |
| Sucres réducteurs | 42 % |

Profil HPLC de l'hydrolysat de farine de maca - Répartition des masses moléculaires :

| Pic HPLC | Masse molaire moyenne (g/mol) | % relatif |
|---|---|---|
| 1 | 1170 | 38,9 |
| 2 | 360 | 29,3 |
| 3 | 180 | 16,2 |
| 4 | 41 | 15,6 |

### EXEMPLE 2 : Activité biologique

### 2-1 Effet sur des fibroblastes humains normaux

### Matériel & Méthode

Cellule : modèle de fibroblastes humains normaux cultivés en monocouche.

Traitement : Les cellules ont été cultivées en l'absence (Contrôle) ou en présence de 0,1% de l'extrait peptidique obtenu à l'exemple 1.

Evaluation du métabolisme cellulaire : L'effet de cet extrait a été évalué par la mesure de l'activité mitochondriale (test au MTT ou 3-(4,5-dimethylthiazole-2-yl)-2,5-diphenyl tetrazolium bromide), tous les jours pendant une semaine.

### Résultats

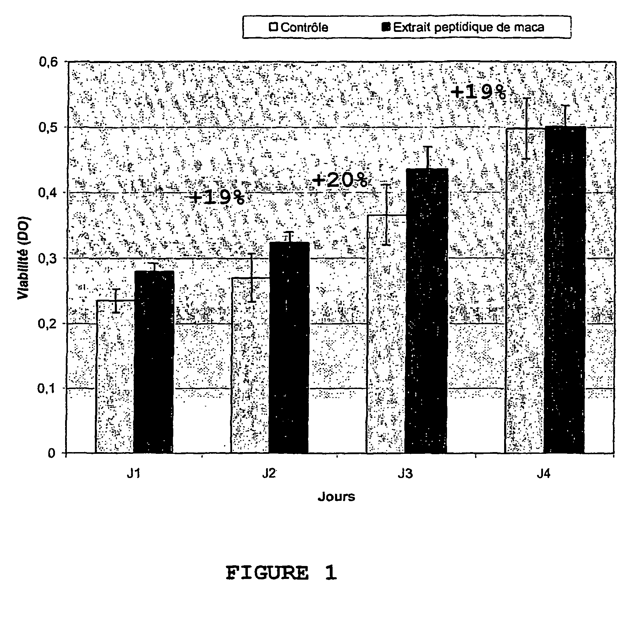

En moyenne, l'extrait peptidique de maca à la dose de 0,1% stimule le métabolisme des fibroblastes dermiques de 20% par rapport aux cellules contrôles non traitées. La figure 1 illustre la viabilité des cellules cultivées en l'absence (Contrôle) ou en présence d'extrait peptidique de maca.

### Conclusion

L'extrait peptidique de maca permet de stimuler le métabolisme cellulaire des fibroblastes dermiques.

### 2-2 Effet sur des fibroblastes humain vieillis artificiellement in vitro

### Modèle d'étude

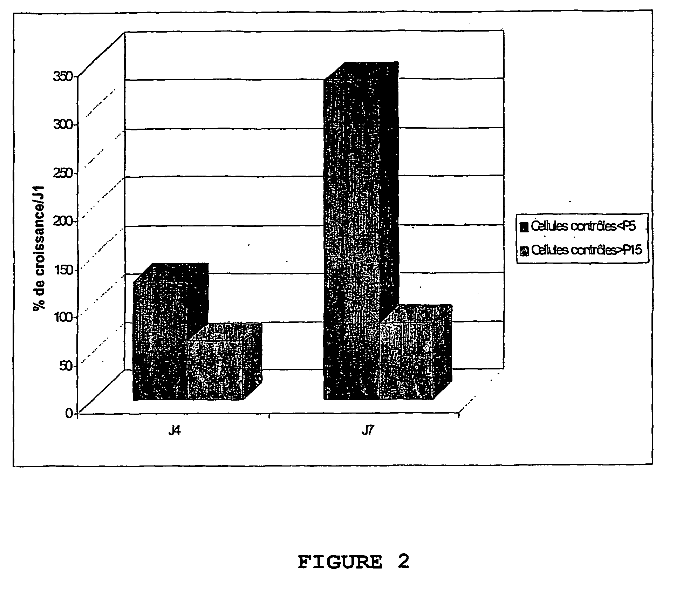

Nous avons utilisé dans cette étude un modèle de fibroblastes cutanés vieillis artificiellement *in vitro*, caractérisé par l'utilisation de fibroblastes issus de chirurgie plastique (femme de 26 ans), et cultivés jusqu'à des passages élevés >p15. En effet, à chaque passage ou dédoublement de population, les fibroblastes:
a) changent d'apparence et sont plus étalés
b) se multiplient beaucoup plus lentement en comparaison avec les mêmes fibroblastes utilisés à des passages <p5 et considérés comme « jeunes » (La figure 2 illustre la comparaison des capacités de réplication (division) de fibroblastes « jeunes » (<p5) et de fibroblastes vieillis (>p15)).

Le vieillissement artificiel *in vitro* ou phénomène de sénescence réplicative a été mis en évidence par Léonard Hayflick en 1961 (Hayflick L and Moorhead PS. The serial cultivation of human diploid cell strains. Exp Cell Res, 25: 585-621 1961). Dans un premier temps, Hayflick a montré que les cellules ne peuvent se diviser qu'un nombre de fois limité dès lors qu'elles sont placées en culture, puis a décrit le lien possible entre sénescence réplicative et vieillissement cellulaire (Hayflick L. The limited lifetime of human diploid cell strains. Exp Cell Res, 37). Les cellules disposeraient d'une horloge interne qui influencerait ou qui limiterait directement leurs capacités de division. Cet arrêt programmé de la division cellulaire pourrait être lié à la perte des télomères (extrémités des chromosomes). Une correspondance acceptable entre *in vivo* (perte d'environ 50 paires de base/dédoublement cellulaire) et *in vitro* (perte d'environ 70 paires de base/dédoublement cellulaire) tend à montrer que le modèle de division cellulaire *in vitro* préfigure ce qui se réalise *in vivo.*

### Résultats

Les cellules ont été cultivées pendant 7 jours en présence ou en l'absence d'extrait peptidique de maca à la dose de 0,01%. La viabilité cellulaire a été mesurée par un test au MTT. Les résultats sont exprimés en % de croissance/au premier de jour de culture (j1) selon la formule : [(DO jX - DO j1)/DO J1] x 100, avec DO = densité optique mesurée à 570 nm; j = jour de culture.

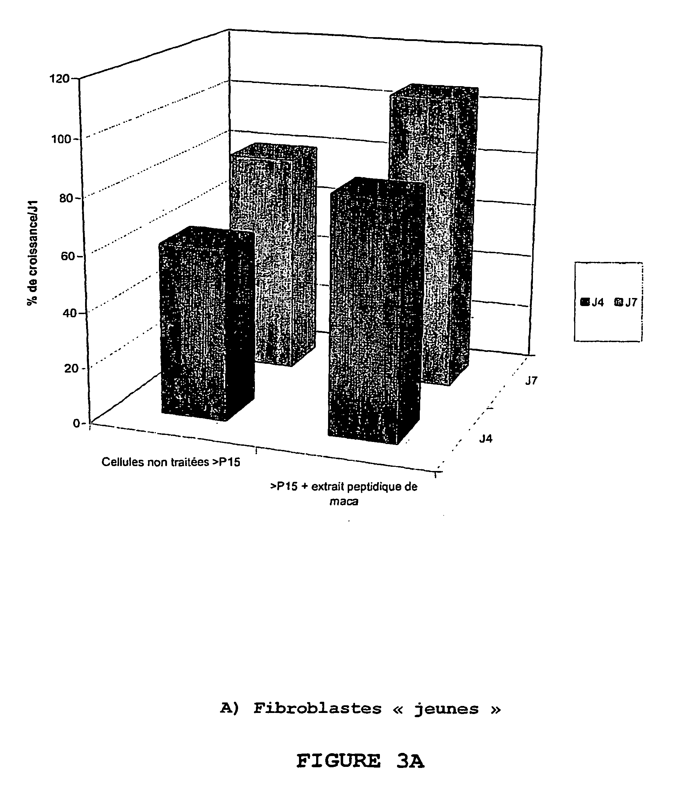

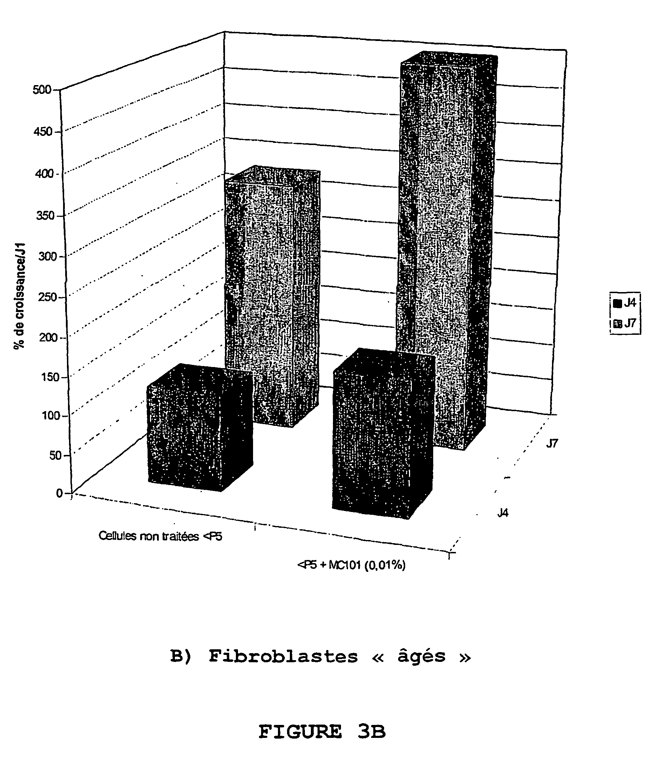

Dans ces conditions expérimentales, l'hydrolysat de maca, à la dose de 0,01%, permet d'augmenter les capacités de division des fibroblastes « jeunes » (passages<5), +35 et 40% respectivement à j4 et j7 (Figure 3A), et des fibroblastes « âgés » (>p15), +26 et 29% respectivement à j4 et j7 (Figure 3B). Les figures 3A et 3B sont annexées.

### Conclusion

L'hydrolysat de maca en stimulant les capacités prolifératives des fibroblastes «âgés» peut donc compenser la diminution de la population cellulaire dermique liée à l'âge et donc s'opposer au vieillissement cutané intrinsèque.

### 2-3 Effet sur la production de peroxydes lipidiques dans des cellules humaines.

Le but de l'étude est d'évaluer les effets de l'extrait peptidiques de maca sur le taux de pexoxydation lipidique (PL) dans des cellules humaines (Jurkat) exposées ou non à une irradiation UVA + UVB. La mesure de PL a été réalisée en utilisant une sonde fluorescente spécifique, par la méthode de cytométrie de flux. Cette méthode présente l'avantage d'une très grande sensibilité en mesurant la fluorescence des cellules individuelles, et cela sur un grand nombre de cellules (10 000 cellules analysées par échantillon).

### Matériels & Méthodes

Les cellules utilisées sont des cellules lymphoïdes humaines Jurkat réparties en plaques de 24 puis à 8.10⁵ cellules/puits.

Le milieu de culture est le RPMI 1640 (Invitrogen 31870-025), à 37°5C et en présence de 5% de CO₂.

Le milieu d'essai est le MEM sans rouge phénol et sans sérum de veau (polylabo 5503401).

Les essais sont menés à l'aide de l'extrait peptidique obtenu à l'exemple 1, dilué deux fois, C'est-à-dire se présentant sous forme d'un extrait à 5% de matière sèche. Une solution S₁ à 2% de cet extrait est préparée en milieu d'essai.

La référence utilisée est l'hydroxyanisole butylé (BHA Sigma ref. B 1253) en solution à 50 µM dans l'éthanol absolu. Les essais sont menés avec une solution S₂ à 100 µM de BHA préparée en milieu d'essai.

La sonde fluorescente est la 5-N-dodecanoyl-aminofluoresceïne (Free. Rad. Biol. Med., 1997, 22, 13-100) en solution à 5µM dans l'éthanol absolu. Les essais sont menés avec une solution S₃ à 1 µM en sonde, préparée en milieu d'essai.

Les cellules humaines sont pré-incubées en milieu de culture, puis lavées en milieu d'essai. Elles sont ensuite incubées en présence de la solution S₁ de l'extrait peptidique, ou la solution S₂ de référence. La solution S₃ de la sonde est ajoutée à chaque lot pour le dosage de PL

Après 45 minutes d'incubation, la sonde fluorescente est éliminée par lavage par le milieu d'essai.

Une partie des lots est ensuite irradiée par des UVB, une autre partie sans irradiation servant de contrôle.

Après 20 minutes d'incubation, les paramètres de fluorescence sont mesurés par cytométrie de flux.

La détermination de la quantité relative de peroxydes lipidiques (PL) est basée sur la mesure de la diminution de fluorescence de la sonde intégrée aux membranes cellulaires. Une augmentation de signal de fluorescence traduit une diminution du taux basal de peroxydes lipidiques membranaires.

Les études sont réalisées en triplicate.

### Résultats

Les résultats obtenus sont exprimés par la valeur de l'intensité de fluorescence et les pourcentages par rapport au témoin sont calculés avec les valeurs de l'intensité de fluorescence.

Les résultats obtenus sont regroupés dans les tableaux suivants:

Quantité intracellulaire relative de peroxydes lipidiques (PL)

| Traitement | Intensité de fluorescence | Moyenne | sd | 1/intensité de fluorescence | % témoin +UV |
|---|---|---|---|---|---|
| Contrôle - | 1.16 | | | | |
| C11/fluor | 1019 | 1.18 | 0.02 | - | - |
| | 1019 | | | | |
| | 530.21 | | | | |
| Contrôle - UV | 463.66 | 506.42 | 37.11 | 0.00197 | 37 |
| | 525.39 | | | | |
| | 198.83 | | | | |
| Témoin + UV | 172.38 | 186.42 | 13.30 | 0.00536 | 100 |
| | 186.04 | | | | |
| | 362.40 | | | | |
| BHA | 368.20 | 374.67 | 16.49 | 0.00267 | 50 |
| 100 µM | 393.42 | | | | |
| | | | | | |
| Solution de | 254.23 | | | | |
| l'extrait de | 252.27 | 248.66 | 8.02 | 0.00402 | 75 |
| l'Exemple 1 à 2% | 239.47 | | | | |

| | | | | | |
|---|---|---|---|---|---|
| P<0,01 | | | | | |

Quantité intracellulaire relative de peroxydes lipidiques (PL) sans UV

| Traitement | Intensité de fluorescence (n=10000 cellules) | Moyenne | sd | 1/intensité de fluorescence | % témoin +UV |
|---|---|---|---|---|---|
| Contrôle - | 1.50 | 1.33 | 0.14 | - | - |
| C11/fluor | 1.24 | | | | |
| | 1.26 | | | | |
| Contrôle - UV | 530.21 | 506 | 37.11 | 0.00197 | 100 |
| | 463.66 | | | | |
| | 525.39 | | | | |
| BHA | 741.05 | 753 | 16.09 | 0.00133 | 67 |
| 100 µM | 745.64 | | | | |
| | 770.93 | | | | |
| MC101 | 716.73 | 705 | 19.45 | 0.00142 | 72 |
| 2% | 682.84 | | | | |
| | 716.34 | | | | |

| | | | | | |
|---|---|---|---|---|---|
| P<0,01 | | | | | |

L'irradiation a diminué de façon significative. L'intensité de fluorescence de la sonde, traduisant la présence de réactions radicalaires au niveau des membranes cellulaires, et donc augmentation de la quantité de PL.

L'antioxydant de référence BHA testé à 100 µM a inhibé significativement la perte de fluorescence due à l'irradiation de 50% par rapport au témoin, en présence d'UV.

L'extrait peptidique selon l'invention a diminué significativement la quantité de peroxydes lipidiques aussi bien en absence d'UV (diminution de 72% par rapport au témoin) qui en présence d'UV (diminution de 75% par rapport au témoin).

### Conclusion

En empêchant la formation de radicaux libres, l'extrait peptidique de maca permet de contrer un des facteurs importants du vieillissement cutané que constitue la formation d'espèces oxygénées réactives.

### EXEMPLE 3: exemple de formulation cosmétique crème anti-âge.

### Crème anti-âge

| Aqua | QSP 100 |
|---|---|
| Isononyl Isononanoate | 7,00 |
| Di-C12-13 Alkyl Malate | 7,00 |
| Isocetyl Stearate | 5,00 |
| Butylene Glycol | 3,00 |
| Extrait peptidique de maca | |
| Aqueux préparé selon l'exemple 1 | 2,00 |
| Dicaprylyl Ether | 2,00 |
| Silanediol Salicylate | 2,00 |
| Arachidyl Alcohol | 1,65 |
| Tromethamine | 1,18 |
| Cetyl Alcohol | 1,00 |
| Glycine | 1,00 |
| Tocopheryl Acetate | 1,00 |
| Behenyl Alcohol | 0,90 |
| Squalane | 0,79 |
| Sodium Citrate | 0,66 |
| PPG-12/SMDI Copolymer | 0,50 |
| Arachidyl Glucoside | 0,45 |
| Parfum | 0,40 |
| Sclerotium Gum | 0,16 |
| Cetearyl Alcohol | 0,13 |
| Citric Acid | 0,11 |
| Sepigel 305* | 0.10 |
| Système conservateur | QS |

| | |
|---|---|
| *produit commercialisé par la société Seppic | |

## Revendications

1. Procédé de préparation d'un extrait peptidique aqueux de maca hydrosoluble à partir de farine de tubercules de maca broyés **caractérisé en ce qu'**il comprend au moins une étape d'hydrolyse enzymatique de la farine de maca mise en suspension dans l'eau avec une amylase et une protéase, une centrifugation pour éliminer les insolubles, une étape d'ultrafiltration de la solution, et une étape de concentration en matière sèche par diafiltration et/ou par évaporation contrôlée jusqu'à l'obtention d'un extrait peptidique aqueux de maca ayant une teneur en matière sèche comprise entre 1 et 300 g/l, une teneur en sucres réducteurs comprise entre 35 et 45% par rapport à la matière sèche et une teneur en azote alpha aminé comprise entre 2 et 70% par rapport à la matière sèche.

2. Procédé de préparation d'un extrait peptidique hydrosoluble de tubercule de maca selon la revendication 1, **caractérisé en ce que** le ratio amylase/protéase varie de 50/50 à 90/10.

3. Procédé selon les revendications 1 à 2, **caractérisé en ce que** l'ultrafiltration présente un seuil de coupure de 10 kDa.

4. Extrait peptidique aqueux de maca susceptible d'être obtenu par le procédé selon l'une quelconque des revendications 1 à 3.

5. Extrait peptidique aqueux de maca selon la revendication 4, **caractérisé en ce qu'**il présente une teneur en matière sèche comprise entre 2 et 10 g/l.

6. Procédé de préparation d'un extrait peptidique solide de maca, **caractérisé en ce que** l'extrait peptidique aqueux selon la revendication 4 ou 5, éventuellement stérilisée est lyophilisé.

7. Extrait peptidique solide de maca susceptible d'être obtenu par le procédé selon la revendication 6.

8. Extrait peptidique solide de maca selon la revendication 7, **caractérisé en ce qu'**il présente la composition en acides aminés suivante (en pourcentage en poids par rapport au poids total d'acides aminés) :
| | |
|---|---|
| Alanine | 5-9% |
| Arginine | 15-20% |
| Acide aspartique | 8-12% |
| Cystine-cysteine | <2% |
| Acide glutamique | 9-15% |
| Glycine | 3-7% |
| Histidine | 1-6% |
| Isoleucine | 2-7% |
| Leucine | 4-9% |
| Lysine | 3-7% |
| Methionine | 1-5% |
| | |
|---|---|
| Phenylalanine | 4,9% |
| Proline | <1% |
| Sérine | 2-8% |
| Thréonine | 1-7% |
| Tyrosine | 1-7% |
| Valine | 4-10% |
| Tryptophane | < 0,5% |

9. Extrait peptidique de maca selon l'une quelconque des revendications 4, 5, 7 ou 8, utile pour la stimulation de la prolifération et la croissance des cellules cutanées et plus particulièrement des fibroblastes.

10. Extrait peptidique de maca selon l'une quelconque des revendications 4, 5, 7 ou 8, utile pour stimuler l'activité mitochondriale des cellules cutanées et plus particulièrement des fibroblastes.

11. Composition cosmétique **caractérisée en ce qu'**elle comprend un extrait peptidique de maca selon l'une quelconque des revendications 4, 5, 7, ou 8 et au moins un excipient cosmétiquement acceptable.

12. Méthode de traitement cosmétique pour prévenir et/ou lutter contre le vieillissement cutané, **caractérisée en ce qu'**elle consiste à appliquer sur la peau une composition selon la revendication 11.

13. Composition pour application sur la peau comprenant un extrait peptidique de maca selon l'une des revendications 4, 5, 7 ou 8 pour utilisation pour lutter contre les agressions extérieures, choisies parmi le soleil, le tabac, la pollution et le stress.

14. Utilisation d'un extrait selon l'une quelconque des revendications 4, 5, 7 ou 8 en tant qu'actif anti-vieillissement.

15. Utilisation selon la revendication 14 pour stimuler le métabolisme cellulaire et notamment des fibroblastes dermiques.

16. Utilisation selon la revendication 14 pour stimuler l'énergie cellulaire.

17. Utilisation d'un extrait selon l'une quelconque des revendications 4, 5, 7 ou 8 en tant qu'actif pour lutter contre la perte de tonicité et/ou d'élasticité de la peau et/ou pour lutter contre l'apparition de taches pigmentaires de sénescence.

## Claims

1. Method for preparing a hydrosoluble aqueous peptide extract of maca from a powder of ground maca tubers, **characterized in that** it comprises at least one step of enzymatic hydrolysis of the maca powder suspended in water with an amylase and a protease, centrifugation to remove insolubles, a step of ultrafiltration of the solution, and a step of concentration into dry matter by diafiltration and/or by controlled evaporation until the obtention of an aqueous peptide extract of maca with a dry matter content of between 1 g/l and 300 g/l, a reducing sugar content of between 35% and 45% with respect to the dry matter and an alpha amino nitrogen content of between 2% and 70% with respect to the dry matter.

2. Method for preparing a hydrosoluble peptide extract of maca tubers as in claim 1, **characterized in that** the amylase/protease ratio varies between 50/50 and 90/10.

3. Method as in claim 1 or 2, **characterized in that** the ultrafiltration has a cut-off threshold of 10 kDa.

4. Aqueous peptide extract of maca able obtainable by using the method in any of claims 1 to 3.

5. Aqueous peptide extract of maca as in claim 4, **characterized in that** it has a dry matter content of between 2 g/l and 10 g/l.

6. Method for preparing a solid peptide extract of maca, **characterized in that** the aqueous peptide extract as in claim 4 or 5, optionally sterilised, is freeze-dried.

7. Solid peptide extract of maca able obtainable by using the method as in claim 6.

8. Solid peptide extract of maca as in claim 7, **characterized in that** it has the following amino acid composition (as a weight percentage with respect to the total weight of the amino acids):
| | |
|---|---|
| Alanine | 5-9% |
| Arginine | 15-20% |
| Aspartic acid | 8-12% |
| Cystine-cysteine | <2% |
| Glutamic acid | 9-15% |
| Glycine | 3-7% |
| Histidine | 1-6% |
| Isoleucine | 2-7% |
| Leucine | 4-9% |
| Lysine | 3-7% |
| Methionine | 1-5% |
| Phenylalanine | 4.9% |
| Proline | <1% |
| Serine | 2-8% |
| Threonine | 1-7% |
| Tyrosine | 1-7% |
| Valine | 4-10% |
| Tryptophane | <0.5% |

9. Peptide extract of maca as in any of claims 4, 5, 7 or 8, which can be used to stimulate the proliferation and growth of skin cells and more particularly of fibroblasts.

10. Peptide extract of maca as in any of claims 4, 5, 7 or 8, which can be used to stimulate the mitochondrial activity of skin cells and more particularly of fibroblasts.

11. Cosmetic composition **characterized in that** it contains a peptide extract of maca as in any of claims 4, 5, 7 or 8 and at least one cosmetically acceptable excipient.

12. Cosmetic treatment method to prevent and/or combat skin ageing, **characterized in that** it consists of applying to the skin a composition as in claim 11.

13. Composition for application on the skin containing a peptide extract of maca as in any of claims 4, 5, 7 or 8 to be used to combat outside aggressions chosen from sun, tobacco, pollution and stress.

14. Use of an extract as in any of claims 4, 5, 7 or 8 as anti-ageing active agent.

15. Use as in claim 14 to stimulate cell metabolism especially of the dermal fibroblasts.

16. Use as in claim 14 to stimulate cell energy.

17. Use of an extract as in any of claims 4, 5, 7 or 8 as active agent to combat loss of tonicity and/or elasticity of the skin and/or to combat the onset of senescence pigment blemishes.

## Patentansprüche

1. Verfahren zur Herstellung eines wasserlöslichen wässrigen Maca-Peptidextrakts ausgehend von Mehl aus zerkleinerten Maca-Knollen, **dadurch gekennzeichnet, dass** das Verfahren mindestens einen Schritt der enzymatischen Hydrolyse des in Wasser suspendierten Maca-Mehls mit einer Amylase und einer Protease umfasst, eine Zentrifugation zur Entfernung unlöslicher Bestandteile, einen Schritt der Ultrafiltration der Lösung, und einen Schritt der Konzentration der Trockenmasse durch Diafiltration und/oder durch kontrollierte Verdampfung bis zum Erhalt eines wässrigen Maca-Peptidextrakts mit einem Trockenmassegehalt zwischen 1 und 300 g/l, einem a Gehalt an reduzierendem Zucker zwischen 35 und 45% bezogen auf die Trockenmasse und einem α-Amino-Stickstoffgehalt zwischen 2 und 70% bezogen auf die Trockenmasse.

2. Verfahren zur Herstellung eines wasserlöslichen Peptidextrakts aus Maca-Knollen gemäß Anspruch 1, **dadurch gekennzeichnet, dass** das Verhältnis Amylase/Protease von 50/50 bis 90/10 variiert.

3. Verfahren nach Anspruch 1 oder 2, **dadurch gekennzeichnet, dass** die Ultrafiltration eine Ausschlussgrenze von 10 kDa aufweist.

4. Wässriger Maca-Peptidextrakt, erhältlich gemäß dem Verfahren nach einem der Ansprüche 1 bis 3.

5. Wässriger Maca-Peptidextrakt nach Anspruch 4, **dadurch gekennzeichnet, dass** er einen Trockenmassegehalt zwischen 2 und 10 g/l aufweist.

6. Verfahren zur Herstellung eines festen Maca-Peptidextrakts, **dadurch gekennzeichnet, dass** der wässrige Peptidextrakt nach Anspruch 4 oder 5, der gegebenenfalls sterilisiert ist, lyophilisiert wird.

7. Fester Maca-Peptidextrakt, erhältlich gemäß dem Verfahren nach Anspruch 6.

8. Fester Maca-Peptidextrakt nach Anspruch 7, **dadurch gekennzeichnet, dass** er die folgende Aminosäurezusammensetzung (in Gew.% bezogen auf das Gesamtgewicht der Aminosäuren) aufweist:
| | |
|---|---|
| Alanin | 5-9% |
| Arginin | 15-20% |
| Asparaginsäure | 8-12% |
| Cystin-Cystein | <2% |
| Glutaminsäure | 9-15% |
| Glycin | 3-7% |
| Histidin | 1-6% |
| Isoleucin | 2-7% |
| Leucin | 4-9% |
| Lysin | 3-7% |
| Methionin | 1-5% |
| Phenylalanin | 4,9% |
| Prolin | <1% |
| Serin | 2-8% |
| Threonin | 1-7% |
| Tyrosin | 1-7% |
| Valin | 4-10% |
| Tryptophan | <0,5% |

9. Maca-Peptidextrakt nach einem der Ansprüche 4, 5, 7 oder 8, verwendbar für die Stimulation der Proliferation und des Wachstums von Hautzellen und insbesondere von Fibroblasten.

10. Maca-Peptidextrakt nach einem der Ansprüche 4, 5, 7 oder 8, verwendbar zum Stimulieren der Aktivität der Mitochondrien der Hautzellen und insbesondere der Fibroblasten.

11. Kosmetische Zusammensetzung, **dadurch gekennzeichnet, dass** sie einen Maca-Peptidextrakt nach einem der Ansprüche 4, 5, 7 oder 8 umfasst und mindestens einen kosmetisch akzeptablen Träger.

12. Kosmetisches Behandlungsverfahren zur Vorbeugung und/oder Bekämpfung der Hautalterung, **dadurch gekennzeichnet, dass** das Verfahren aus dem Auftragen einer Zusammensetzung nach Anspruch 11 auf die Haut besteht.

13. Zusammensetzung zum Auftragen auf die Haut, umfassend einen Maca-Peptidextrakt nach einem der Ansprüche 4, 5, 7 oder 8 für die Verwendung zur Bekämpfung von äußeren Einflüssen, ausgewählt aus Sonne, Tabak, Verschmutzung und Stress.

14. Verwendung eines Extrakts nach einem der Ansprüche 4, 5, 7 oder 8 als Anti-Aging-Wirkstoff.

15. Verwendung nach Anspruch 14 zum Stimulieren des Zellstoffwechsels, insbesondere der Fibroblasten der Haut.

16. Verwendung nach Anspruch 14 zum Stimulieren der Zellenergie.

17. Verwendung eines Extrakts nach einem der Ansprüche 4, 5, 7 oder 8 als Wirkstoff zum Bekämpfen des Verlustes des Hauttonus und/oder der Hautelastizität und/oder zum Bekämpfen des Erscheinens von altersbedingten Pigmentflecken.
